# EUROPEAN PATENT APPLICATION

(11) **EP 2 862 629 A1**
(43) Date of publication of application: **22.04.2015**
(21) Application number: 13188709.3
(22) Date of filing: 15.10.2013
(51) Int. Cl.: B01J 37/08, B01J 37/12, B01J 37/16, B01J 23/02, B01J 23/28, B01J 23/30, B01J 23/92, B01J 35/00, B01J 35/10, C07C 6/04, B01J 35/02

(54) **Catalyst and process for olefin metathesis reaction**

(71) Applicant: Borealis AG, 1220 Vienna (AT)
(72) Inventor: Stoyanova, Marina, 12207 Berlin (DE); Kondratenko, Evgenii, 18057 Rostock (DE); Linke, David, 10318 Berlin (DE); Ernst, Eberhard, 06667 Weißenfels (DE)
(74) Representative: Morawski, Birgit

(57) **Abstract**

The present invention relates to magnesium oxide (MgO) for use in olefin metathesis with defined physical properties, a catalyst for olefin metathesis comprising said MgO and a process for olefin metathesis using said catalyst.

## Description

The present invention relates to magnesium oxide for use in olefin metathesis according to claim 1, the use of said magnesium oxide according to claim 8, a catalyst for olefin conversion according to claim 9, and a process for obtaining an olefin according to claim 15.

### Description

Butenes are the C₄H₈ mono-olefin isomers such as 1-butene, cis-2-butene, trans-2-butene and iso-butene (2-methylpropene). If it is not specifically mentioned, cis-2-butene, trans-2-butene are also called as 2-butene within the frame of the present invention. The sum of cis-2-butene, trans-2-butene, and 1-butene is denoted as n-butenes. Butenes are almost always commercially produced as by-products in a petroleum refinery by cracking processes or by catalytic ethene dimerisation. Butenes can be used for multiple purposes like in the manufacture of polymers and other chemicals like insecticides, antioxidants, adhesives, sealants or elastomers.

The use of 2-butenes for the production of propene has gained industrial importance in the last decades. The synthesis of propene using 2-butenes as starting material is based on the metathesis reaction. Hereby, 2-butene is converted in the presence of ethene to propene according to the following overall reaction scheme:

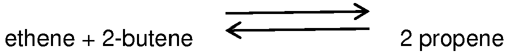

This reaction occurs typically in the presence of a catalyst comprising metal oxide of the group 6 or 7 of the periodic system of the elements (PSE). Typical active components of catalysts used in olefin metathesis are tungsten oxide supported on silica (US 3,365,513), rhenium oxides or molybdenum oxides supported on alumina or silica-alumina (US 4,547,617; US 6,281,402).

Various modifications and improvements of the metathesis catalysts have been described. The physical mixing of the metathesis catalyst with an isomerisation catalyst for shifting the double bond in 1-butene to 2-butene has been proven to increase the overall propene production yield (US 3,865,751; US 3,915,897; US 4,575,575).

Typical double bond isomerisation catalysts include basic metal oxides as for instance magnesium oxide or calcium oxide, which can be admixed with the metathesis catalyst. The use of magnesium oxide (MgO) as a co-catalyst enables reduction of the reaction temperature to 250-300°C from approximately 400°C for pure silica supported tungsten oxide (WO₃/SiO₂). The weight ratio of magnesium oxide to WO₃/SiO₂ is in the range of 0.1-20. Magnesium oxide has the function to isomerise 1-butene to 2-butene and/or 2-butene to 1-butene (this isomerisation is an equilibrium reaction).

Besides its ability to act as an isomerisation catalyst magnesium oxide has also been known for its ability to remove or destroy traces of contaminants from the olefin feed that are detrimental to metathesis catalysts, in particular when used as a "guard bed" (J. Mol. Cat. 1985, 28:117-131). Magnesium oxide can be for instance arranged on top of a composition comprising the metathesis catalyst and an isomerisation catalyst (US 2010/0056839 A1, US 2010/167911 A1). Here the optimal catalyst performance is combined with the guard pre-bed function to remove poisons and the isomerisation of 1-butene to 2-butene and/or 2-butene to 1-butene. When applying this approach a technical metathesis reactor is typically filled with a mixture of MgO and WO₃/SiO₂ as catalyst main-bed and a MgO as pre-bed upstream of the main bed.

MgO must be activated to achieve the desired properties. Different activation procedures have been described. According to US 4,071,471 magnesium oxide is activated by heating in a flowing stream of an oxygen containing gas for about 1 to 30 hrs at 300 to about 550°C. Another activation method comprises the treatment of magnesium oxide with carbon monoxide or hydrogen in the range of about 250 to 650°C for about 0.1 to 4 hours (US 3,546,313). It is also possible to heat the pre-activated catalyst, which was pre-activated according to one of the above described methods, in a stream of an inert gas in the temperature range up to 600 °C (Chemical Reviews, 95 (3), (1995) 537-558).

Magnesium oxide can be produced from various raw materials, for example, by the calcination of magnesium carbonate or magnesium hydroxide or by the treatment of magnesium chloride with lime followed by heating. It is known to prepare MgO from Mg(OH)₂ (magnesium hydroxide) which is available in form of the naturally occurring brucite mineral. Mg(OH)₂ may be calcinated at elevated temperature in air or in vacuum, whereby only MgO obtained by calcination of Mg(OH)₂ in vacuum shows any isomerisation activity of 1-butene (Proceedings of the International Congress on Catalysis 1973, 1, 233-242). It also should be pointed out that Mg(OH)₂ may contain an undefined amount of carbonate, in particular due to its highly disturbed lattice structure.

According to the Bulletin of the Chemical Society of Japan (49(4), (1976) 969-72) MgO was obtained by heating Mg(OH)₂ or (MgCO₃)₄•Mg(OH)₂•5H₂O (Mg carbonate hydroxide) in vacuum at different temperatures in order to investigate the effect of the precursor material and treatment parameters on the surface area of MgO and its structural and catalytic properties in alkylation of phenol with methanol and in isomerisation of butenes. It was shown that the precursor did not influence the activity and the selectivity (ratio of cis-2-butene to trans-2-butene) of MgO in 1-butene isomerisation. However, the activity and the selectivity for the isomerisation of cis-2-butene in respect to the ratio of trans-2-butene to 1-butene were in case of MgO prepared from Mg(OH)₂ about 4 times higher than in case of MgO prepared from (MgCO₃)₄•Mg(OH)₂•5H₂O.

The optimization of the catalyst performance is of general interest in various technical processes. Particularly, an increase in conversion of feed components, product selectivity and yield has a strong impact on the process economics.

The catalyst performance depends to a large extent on the preparation procedure. The general parameters of the activation process of isomerization catalysts, e.g. MgO, are well known (see state of the art), but nothing is known about the relation between activation parameters, crystalline structure and catalytic properties in the case of the catalyst preparation for the propene production by cross-metathesis of ethene and 2-butenes.

It is therefore an object of this invention to provide an isomerisation catalyst for the metathesis of olefines which shows improved activity and selectivity compared to the presently known compounds.

This and other objects of the invention were solved by a Magnesium oxide and catalyst for olefin conversion with the features of the claims.

Accordingly, magnesium oxide (MgO) typically used in olefin metathesis is characterized by specific physical properties. The present MgO has a specific surface area BET of 50 to 300 m²/g; a crystallite size of 5 to 25 nm; a total pore volume of 0.1 to 0.5 cm³/g; and a maximum of pore size distribution of 5 to 15 nm.

Such MgO is preferably obtained by calcination of Magnesium carbonate hydroxide of the chemical formula of (MgCO₃)₄•Mg(OH)₂•5H₂O in the presence of an oxygen-containing gas, in particular in the presence of air such as in an air flow. Thus, the present MgO is obtained from a precursor compound with a defined amount of carbonate.

The present MgO shows surprisingly an increased activity, in particular isomerisation activity and time on stream activity, when combined with a metathesis catalyst in the olefin conversion, in particular in the cross-metathesis of ethene and 2-butene, compared to MgO conventional prepared from Mg(OH)₂ as shown in the Examples below. Thus, an improved metathesis catalyst with better time-on-stream activity (lower deactivation rate) and improved time-on-stream isomerisation properties is provided due to optimised MgO properties.

The increase of activity and stability was not expected since so far MgO prepared from (MgCO₃)₄•Mg(OH)₂•5H₂O by calcination in air did not show any isomerisation activity for 1-butene (Proc. Int. Congr. Catal, (1972), 233-243); solely MgO obtained by calcination of (MgCO₃)₄•Mg(OH)₂•5H₂O in vacuum revealed any isomerisation activity. It was believed that the inactivity of MgO obtained from (MgCO₃)₄•Mg(OH)₂•5H₂O in air was caused by the coverage of the active sites of MgO with small amounts of water and carbon dioxide when calcinated in air.

In an embodiment the present MgO has a specific surface area BET of 80 to 150 m²/g, preferably 100 to 120 m²/g. A typical BET is about 105 to 115 m²/g.

In a further embodiment the present MgO has a crystallite size of 10 to 20 nm, preferably 10 to 15 nm, whereby a typical crystallite size is 13-14 nm.

In another embodiment the present MgO has a total pore volume of 0.2 to 0.4 cm³/g, preferably 0.3 to 0.4 cm³/g, whereby a typical value is 0.35-0.36 cm³/g.

It is furthermore preferred if the present MgO has a maximum of pore size distribution between 7 and 10 nm, preferably between 8 and 9 nm.

It is of an advantage if the present MgO is obtained by calcination of (MgCO₃)₄•Mg(OH)₂•5H₂O in oxygen-containing gas at temperatures between 300 °C and 700 °C, preferably 400 °C and 600 °C, most preferably 450 °C and 550 °C.

The present MgO is used as catalyst for isomerisation of olefines, in particular 1-butene and/or 2-butenes (cis- or trans-2-butene). The isomerisation activity of the present MgO and its guard property is in particular prevalent when combined with a suitable metathesis catalyst.

Accordingly, a catalyst (main catalyst bed) is being provided, in particular suitable for olefin conversion technology comprising metathesis, which comprises a) at least one first catalyst component comprising a metathesis catalyst, and b) at least one second catalyst component comprising a catalyst for double bond isomerisation, wherein the catalyst for double bond isomerisation is the present MgO obtained by calcination of Magnesium carbonate hydroxide of the formula (MgCO₃)₄•Mg(OH)₂•5H₂O in the presence of an oxygen-containing gas. The first and second catalysts are physically mixed with each other.

In a further embodiment the metathesis catalyst comprises metal oxides from metals of group 6 and 7 of the PSE, in particular tungsten oxide, molybdenum oxide and/or a precursor thereof, which are the active components and are deposited on at least one inorganic carrier. The most preferred metal oxide is tungsten oxide.

Preferably, at least one inorganic carrier is selected from a group comprising silica, alumina, silica-alumina or aluminium phosphate. The inorganic carrier can contain at least about 0.1 wt% and up to 40 wt% of the active components. Amounts between 1 to 30 wt% are preferred, whereby amounts between 2 to 15 wt% are mostly preferred.

The metathesis catalyst may further comprise at least one oxide of a metal of group I of the PSE or a precursor thereof as for instance comprising oxides, hydroxides, carbonates, bicarbonates, nitrates, acetates of sodium or potassium or mixtures thereof. Especially preferred are the hydroxides of sodium and potassium. The amount of these modifying compounds can be between 0.01 and 10 wt%, preferably between 0.1 and 10 wt% with respect to the metathesis catalyst.

It is further possible that the metathesis catalyst undergoes a pre-treatment with at least one oxide of a member of group 1 of the PSE or a precursor thereof. For example it is preferred if silica supported tungsten oxide is used as metathesis catalyst it undergoes a pre-treatment with potassium hydroxide.

The BET surface area of the metathesis catalyst is at least > 10 m²/g, preferably at least > 50 m²/g and mostly preferably at least ≥ 100 m²/g.

The particle size of the metathesis catalyst depends on the reactor size. When applied as powder like for instance in lab size reactors, the typical particle size of the metathesis catalyst is between 0.3-0.7 mm. When used in larger reactors like for instance technical reactors the particle size is in the range between 1 and 10 mm, preferably between 1 and 8 mm, most preferably between 1 and 5 mm.

The present catalyst can then be prepared by admixture of the present MgO as double bond isomerisation catalyst and the metathesis catalyst. The catalysts are preferably mixed in form of powders, pellets or extrudates.

The amount of the isomerisation catalyst is preferably in excess of the amount of the metathesis catalyst. However, the present MgO used as isomerisation catalyst can also be used in lower amounts. In an embodiment the catalyst composition or mixture comprises the at least isomerisation catalyst component and the at least one metathesis catalyst component in a ratio between 5:1 and 1:1, preferably in a ratio 4:1 and 2:1, most preferably in a ratio of 3:1. It is important to note here that the weight ratio of isomerisation catalyst to metathesis catalyst does not show any influence on the catalyst performance or activity and yield.

It is also possible that the present MgO is additionally arranged as a pre-bed (catalyst pre-bed) upstream of the catalyst mixture of metathesis catalyst and isomerisation catalyst. In this case of a catalyst bed configuration the present MgO as pre-bed may be located immediately upstream and/or directly as a top layer on the top surface of the main catalyst bed of the mixture of metathesis catalyst and isomerisation catalyst.

In general it is also possible to use non-modified and commercially available MgO as pre-bed. Thus, a catalyst bed configuration is conceivable comprising a as main catalyst bed a metathesis catalyst and the present modified MgO and a catalyst pre-bed comprising a non-modified MgO.

It is of an advantage if the mass ratio of the pre-bed MgO and the main catalyst bed being the mixture of metathesis catalyst and isomerisation catalyst is between is between 1:10 and 3:1, preferably between 1:6 and 2:1, most preferably between 1:4 and 1:2.

The pre-bed made of the present MgO may be used for the purification of olefin streams. This purification is based on the removal of traces of moisture, carbon dioxide and other polar compounds by adsorption. These compounds act as poisons for the catalyst when entering the reactor. Said compounds are adsorbed on the metathesis catalyst components particular on MgO and form acidic centres which form the source for coke formation. Subsequently, the coke covers the active sites resulting in catalyst deactivation. The result of this process is visible as decline of the yield/ conversion curve over the reaction time (tos). Thus, when using the present MgO pre-bed the olefin streams are purified before entering the main catalyst mixture.

The present catalyst (main catalyst bed) being a mixture of metathesis catalyst and present MgO as isomerisation catalyst is activated before the actual metathesis reaction of olefins. Such an activation process comprises the steps of:
a) heating the catalyst in an inert gas atmosphere to a temperature between 300 °C and 500 °C, preferably 400 °C;
b) oxidizing the catalyst in an oxygen containing atmosphere e.g. such as air at temperatures between 400 °C and 600 °C, preferably 400 °C and 550 °C;
c) reducing the catalyst in a hydrogen containing atmosphere at temperatures between 300 °C and 500 °C, preferably at 400 °C,
d) again heating the catalyst in an inert gas atmosphere at temperatures between 400 °C and 600 °C, preferably 400 °C and 550 °C; and
e) Subsequent cooling down the catalyst in an inert gas atmosphere.

In the course of the above activation of the catalyst (main catalyst bed) at first the MgO is activated followed by activation of the metathesis catalyst, whereat water is formed. Said water in turn may partially deactivate MgO the activity thereof being finally restored.

In a typical embodiment of the activation procedure the catalyst is heated starting at room temperature at a heating rate of 5 °C/min until an end temperature e.g. of about 400°C is reached and is held at this temperature for about 2 hours.

In the next step the catalyst is oxidized in air, wherein the start temperature may be 400°C and the end temperature may be 525°C. The heating rate is about 5°C/min during the oxidation and the holding time at the end temperature may be about 2 hours.

Subsequently the oxidized catalyst is cooled down in an inert gas atmosphere, such as nitrogen gas atmosphere from the oxidation temperature of e.g. 525°C to 400°C and is held at the latter temperature for about 0.5 h. The reduction of the catalyst is carried out in a gas mixture of nitrogen and hydrogen with a ratio of about 80:20, preferably 70:30 at e.g. about 400°C for about 0.5 -1 h, preferably for about 0.5 h. Following the reduction the catalyst is now purged with nitrogen at 400°C for about 0.5-1h, preferably for about 0.5 h.

The catalyst reduction is followed by a further heating step in a flow of an inert gas, such as nitrogen. Here, desorption of adsorbed impurities from the catalyst surface takes place. The desorption step may last 10-20 h, preferably 14-16 h. During this time the temperature may be raised from about 400°C to about 550°C with a heating rate of about 5°C/min. Finally, the catalyst is cooled down in an inert gas atmosphere, e.g. nitrogen gas.

It is to be understood that the process conditions for activating the main catalyst bed as described are above are solely exemplarily and depend on the size of the catalyst bed and reactor size. The process conditions should be adapted accordingly. This is however part of the routine work of a person skilled in the art, such as a process engineer.

The present catalyst mixture is preferably used in a reactor and in a process for the conversion of at least two olefins by metathesis. It is in particular preferred if the present catalyst mixture is used for the conversion of ethene and at least one butene (e.g. 2-butene) to propene by metathesis.

The catalyst mixture is preferably part of a fixed-bed reactor. Basic types of catalytic fixed bed reactors are the adiabatic fixed-bed reactor and the isothermal fixed bed reactor. The adiabatic fixed-bed reactor is preferred for technical processes. The catalyst is usually provided in the fixed-bed reactor in form of random packings of powders, pellets or extrudates, for instance of catalytic pellets.

Typically the reactor is a packed fixed-bed reactor, which is widely used for gas solid reactions.

In an embodiment the reactor has a length to diameter ratio (I/d ratio) between 1 and 15, preferably between 1 and 10, most preferably between 1 and 5, even more preferably between 1.5 and 3.5.

The catalyst mixture and the reactor are used in a process for obtaining an olefin, in particular propene, by metathesis comprising the steps of
- feeding at least two olefins as starting material to a reactor, in particular a fixed bed reactor, comprising at least one catalyst mixture of metathesis catalyst and the present MgO and
- converting the at least two olefins at a pressure between 1 to 50 bar, in particular between 10 to 30 bar, at a temperature between 100 and 600 °C, in particular between 250 and 500 °C to at least one new olefin by metathesis.

The metathesis reaction is preferably performed at a weight hourly space velocity (WHSV) in the range between 1 and 100 h⁻¹, preferably between 1 and 50 h⁻¹, more preferably between 1 and 10 h⁻¹ (the WHSV values are referring to the main catalyst bed and the fed 2-butene).

In an embodiment the one of the at least two olefins used as starting material comprises at least two carbon atoms, such as ethene, and the second of the at least two olefins used as starting material comprises at least four carbon atoms, such as a 2-butene. The mole ratio between said olefin comprising at least two carbon atoms and the olefin comprising at least four carbon atoms can be between 1 and 20, preferably 1 and 10, mostly preferably between 1 and 5.

The at least two olefins may be supplied to the reactor as a mixed stream or in form of separated streams. When using 2-butene as starting material, the butene component may be supplied as cis- or trans-2-butene or mixtures thereof. A technical 2-butene stream may contain additional small amounts of n-butane, isobutane, isobutene, 1-butene. In some embodiments the mixed C4 stream is pre-treated to increase the 2-butene content in the feed for the metathesis reaction. If a crude C4 cut from an e.g. naphtha cracker is used compounds like 1,3-butadiene, allene or acetylenes have to be removed by a selective hydrogenation step. The olefin mixture is then contacted with the catalyst bed, whereby the olefins contact at first the catalyst pre-bed and then the main catalyst bed. In the catalyst pre-bed isomerisation as wells as purification of the feed occur. When entering the main catalyst bed comprising the metathesis catalyst and the isomerisation catalyst, isomerisation in particular of 1-butene to 2-butene and the synthesis of propene from ethene and 2-butene occur. Besides propene also other reaction products can be formed such as for example C5-C6 olefins.

The process may be carried out by contacting the olefins with the catalyst in the liquid phase or the gas phase depending on structure and molecular weight of the olefins used as starting material, the catalyst used and/or the reaction conditions applied such as pressure, temperatures etc.. Diluents such as saturated aliphatic hydrocarbons, such as methane, ethane, propane, butane and/or inert gases like nitrogen or argon might be suitable. In any case, the presence of deactivating substances like water or oxygen should be avoided.

The metathesis catalyst is very sensitive to impurities in the feed stream. Such feed poisons are, for example, strong polar or protic compounds such as N-, O-, S- and halogen comprising compounds or carbon oxide derivatives (oxygenates). Typical examples are water, alcohols, ethers, ketones, aldehydes, acids, carbon dioxide, carbon monoxide, carbon oxide sulfide and the like. The consequences are reduced catalyst activity and shortened cycle times. Therefore the feed stream must be purified by passing it through suitable adsorbents before feeding to the reactor.

It is also possible to conduct the reaction in the presence of hydrogen (EP 1854776 A1).

The effluent from the metathesis reactor can be sent to a separation system for separating the product(s) from unreacted feed components. For instance, the products of the separation system may include ethene, propene, C4- and C5-compounds. The propene separated from the reaction stream is characterised by a high purity. The ethene and C4 olefins may be recycled back to the metathesis reactor or to a pre-treatment stage.

The present invention is further explained in more detail by the means of the following examples with reference to the Figure. It shows:
- Figure 1: a diagram illustrating the time-on-stream a) conversion of n-butene and b) yield of propene in ethene and 2-butene metathesis for an embodiment of a catalyst according to the invention;
- Figure 2: a diagram illustrating the time-on-stream a) conversion of n-butene and b) yield of propene in ethene and 2-butene metathesis using a catalyst containing MgO prepared from Mg(OH)₂;
- Figure 3: a diagram illustrating the time-on-stream a) conversion of n-butene and b) yield of propene in ethene and 2-butene metathesis using a catalyst containing commercial MgO; and
- Figure 4: a diagram illustrating the time-on-stream mol fractions of a) 1-butene and b) cis-2-butene obtained under standard reaction conditions using MgO acording to the invention (labelled as MgO_{L1}); MgO prepared from Mg(OH)₂ (labelled as MgO_{L2}) and commercial MgO (labelled as MgO_{c}).

### Example 1: Inventive example

MgO_{L1} (MgO according to the invention) was prepared by calcination of (MgCO₃)₄•Mg(OH)₂•5H₂O at 550 °C for 16 h in an air flow. The results of MgO_{L1} characterisation by BET and XRD are given in Table 2 below.

(WOₓ/SiO₂)_{L1} was prepared by wet impregnation of SiO₂ (Aerolyst® 3038, Evonik) with a solution of ammonium metatungstate hydrate (Aldrich 99.99%, trace metals basis) and potassium hydroxide (Merck). The tungsten (calculated for WO₃) and potassium (calculated for K₂O) loadings were set to approximately 7 and 0.2 wt.%, respectively, as described in US 4,575,575.The dried catalyst precursors were calcinated in a muffle oven with circulating air flow at 538 °C for 8 h.

The calcined (WOₓ/SiO₂)_{L1} and MgO_{L1} powders were then pressed, crushed and sieved to obtain particles of 315-710 µm.

The catalyst was then activated according to activation steps as outlined in Table 1.

**Table 1: activation procedure for catalyst**

| Activation steps | Tₛₜₐᵣₜ / °C | Tend / °C | Heating rate / °C/min | Holding time at Tend / h |
|---|---|---|---|---|
| Heating in N₂ from room temperature | 25 | 400 | 5 | 2 |
| Oxidation in air | 400 | 525 | 5 | 2 |
| Cooling down in N₂ | 525 | 400 | 2 | 0.5 |
| Reduction in N₂/H₂=70/30 | 400 | 400 | | 0.5 |
| Purge with N₂ | 400 | 400 | | 0.5 |
| Desorption in N₂ | 400 | 550 | 5 | 16 |
| Cooling down in N₂ | 550 | 300 | | |

Pure MgO_{L1} was tested for trans-2-butene isomerization in presence of ethene at 300 °C. The cross-metathesis of ethene and trans-2-butene was also investigated at 300 °C but using a catalysts bed consisting of MgO_{L1} pre-bed and a mixture of MgO_{L1} and (WOₓ/SiO₂)_{L1}, *i.e.* MgO_{pre-bed}/(MgO:(WOₓ/SiO₂)=3:1)=0.25. Ethene and trans-2-butene were extra purified using molsieve 3A. An additional triple gas filter cartridge (Oxygen, Moisture and Hydrocarbon trap, Restek) was used to remove oxygen, moisture and hydrocarbons form nitrogen, hydrogen, and hydrogen mixtures. A standard reaction feed consisted of C₂H₄, trans-2-C₄H₈, and N₂ (10 vol.%) with a C₂H₄/trans-2-C₄H₈ ratio of 2.5. The weight hourly space velocity (WHSV related to the main catalyst bed, namely MgO/WOₓ-SiO₂-mix mass) was set to 1.9 h⁻¹ with respect to co-fed trans-2-butene (standard reaction conditions).

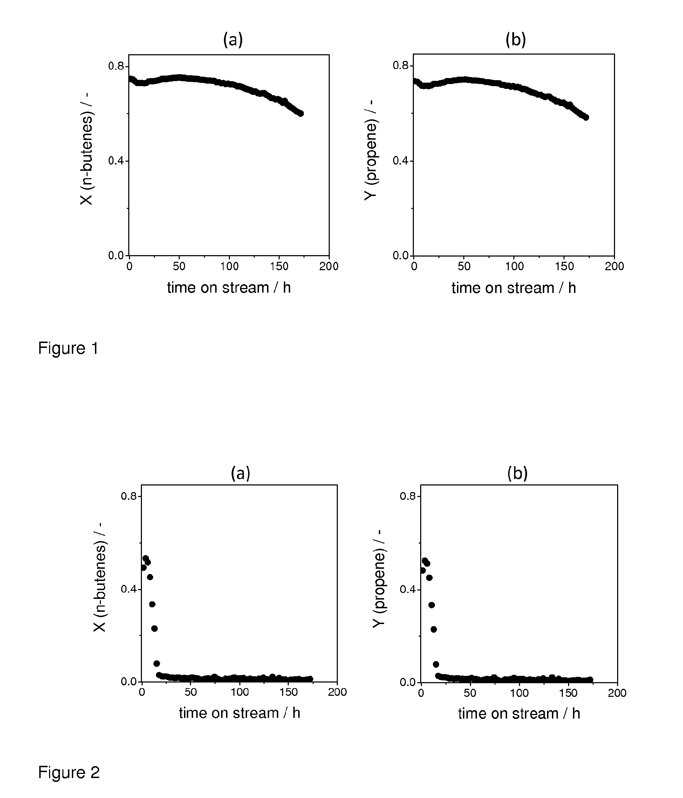

The results of metathesis and isomerization tests are shown in Figure 1. The diagram in figure 1 shows the time-on-stream (a) conversion of n-butenes and (b) yield of propene over MgO_{L1}/(MgO_{L1}:(WOₓ/SiO₂)_{L1})=0.25) under standard reaction conditions. As deducible from the diagrams of Figures 1 it becomes apparent that MgO_{L1} prepared from (MgCO₃)₄•Mg(OH)₂•5H₂O by calcination in air shows a very good time-on stream stability.

### Example 2: Comparative example

MgO_{L2} was prepared by calcination of Mg(OH)₂ at 550°C for 16 h in an air flow. The results of MgO_{L2} characterisation by BET and XRD are provided in Table 2 below.

(WOₓ/SiO₂)_{L2} was prepared similarly to (WOₓ/SiO₂)_{L1} (see Example 1) but using SiO₂ (Davisil™, Aldrich) instead of SiO₂ (Aerolyst® 3038, Evonik). Both catalytic materials were tested as described in Example 1. It should be noted that the two support materials Aerolyst® 3038 and Davisil™ have the same surface properties and texture.

The results of metathesis and isomerization tests using this catalytic preparation are shown in Figure 2. The diagrams of Figure 2 show that the overall stability of MgO_{L2} is dramatically reduced in comparison to experiment with MgO_{L1}.

### Example 3: Comparative example

Commercial MgO and WOₓ/SiO₂, were used. They are denoted as MgOc and (WOₓ/SiO₂)_{C}, respectively. The results of MgOc the characterization by BET and XRD are provided in Table 2 below. Both catalytic materials were tested as described in Example 2.

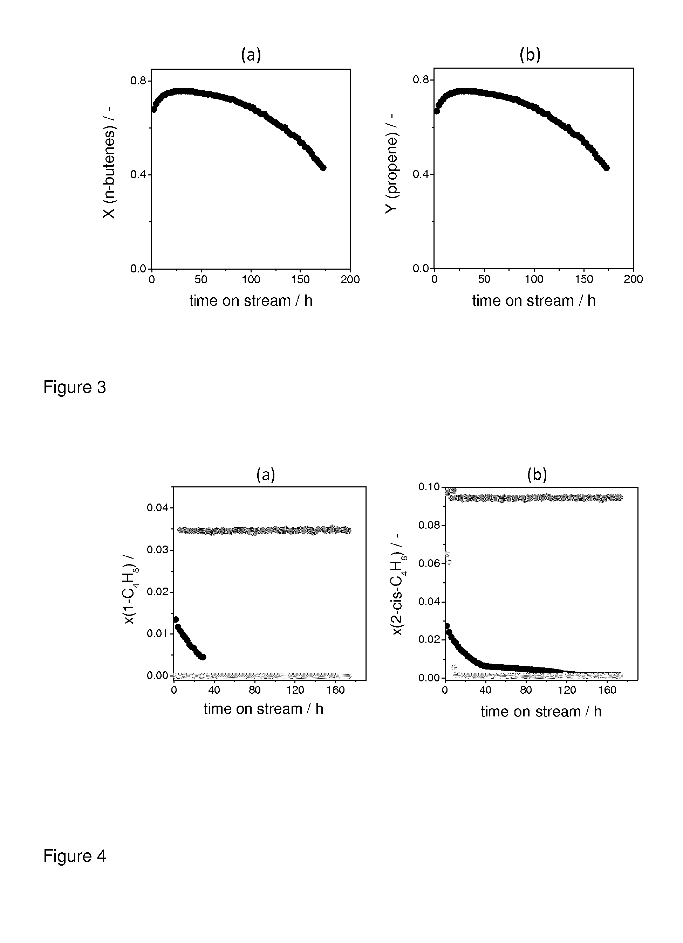

The results of metathesis and isomerization tests are shown in Figure 3. The diagrams of Figure 3 reveal that the overall stability of a commercial available MgO_{c} is lower than of MgO_{L1}.

Figure 4 shows time on stream profiles of the effluent molar fractions of 1-butene and cis-2-butene formed overMgO_{L1} according to the invention, MgO_{L2} obtained from Mg(OH)₂ and commercial MgO_{c}. This direct comparison and supports the results shown in Figures 1-3. It is apparent that the amount of butene converted over time are dramatically improved when using the more stable MgO_{L1}.

### Example 4: Bulk and surface properties of differently originated MgO

**Table 2: List of referenced types of MgO including bulk and surface properties**

| MgO denotation | Precursor | BET [m²/g] | Total pore volume [cm³/g] | Max. of pore size distribution [nm] | Crystallite size [nm] |
|---|---|---|---|---|---|
| MgO_{c} | commercial | 34 | 0.151 | 4.4-5 | 17.9 |
| MgO_{L1} | (MgCO₃)₄•Mg(OH)₂•5H₂O (CAS No 39409-82-0), Acros Organics | 109 | 0.356 | 8-8.4 | 13.3 |
| MgO_{L2} | Mg(OH)₂ (CAS No 1309-42-8), Fluka | 15.5 | 0.189 | n.d. | >100 |

It becomes clear from the above results shown in figures 1-4 that MgO_{L1} is the best promoter or co-catalyst for the metathesis catalyst WO₃-SiO₂. Especially the time-on-stream stability which is characterised by the conversion after e.g. 150 hours is clearly improved. The material has also a better isomerisation activity. The outstanding promoter properties are a result of the combination of the higher specific BET surface area, the higher maximal pore size and the low crystallite size.

The methods used for determining the catalyst properties are standard methods.

### S_{BET} - specific surface area

Nitrogen physisorption at-196 °C on BELSORP-mini II setup (BEL Japan, Inc.) was employed to determine specific surface areas (SBET). The pore size distribution and total pore volume were obtained using the BJH method. The samples were exposed to vacuum (2 Pa) and then heated at 250°C for 2 h before the measurements.

### X-ray diffraction (XRD) - crystallite size

X-ray diffractograms of freshly calcined MgO were recorded in the Bragg angle (2θ) range from 5 to 65° at a rate of 0.01 ° s-1 on a STOE Stadi P setup using Cu Kα radiation (λ = 0.154 nm). The phase identification was carried out basing on the ICDD database.

## Claims

1. Magnesium oxide (MgO) for use in olefin metathesis
**characterized by**
- a specific surface area BET of 50 to 300 m²/g;
- a crystallite size of 5 to 25 nm;
- total pore volume of 0.1 to 0.5 cm³/g; and
- a maximum of pore size distribution of 5 to 15 nm.

2. Magnesium oxide according to claim 1, **characterized by** a specific surface area BET of 80 to 150 m²/g, preferably 100 to 120 m²/g.

3. Magnesium oxide according to claim 1 or 2, **characterized by** a crystallite size of 10 to 20 nm, preferably 10 to 15 nm.

4. Magnesium oxide according to one of the preceding claims, **characterized by** a total pore volume of 0.2 to 0.4 cm³/g, preferably 0.3 to 0.4 cm³/g.

5. Magnesium oxide according to one of the preceding claims, **characterized by** a maximum of pore size distribution of 7 to 10 nm, preferably of 8 to 9 nm.

6. Magnesium oxide according to one of the preceding claims, **characterized in that** it is obtained from magnesium carbonate hydroxide of the formula (MgCO₃)₄•Mg(OH)₂•5H₂O by calcination in the presence of an oxygen-containing gas.

7. Magnesium oxide according to claim 6, **characterized in that** the calcination is conducted at temperatures between 300 °C and 700 °C, preferably 400 °C and 600 °C, most preferably 450 °C and 550 °C.

8. Use of magnesium oxide according to one of the preceding claims as catalyst for isomerisation of olefins, in particular 1-butene and/or 2-butenes.

9. Catalyst for conversion of olefins comprising a mixture of
a) at least one first catalyst component comprising a metathesis catalyst, and
b) at least one second catalyst component comprising magnesium oxide according to one of the claims 1 to 7 as catalyst for double bond isomerisation.

10. Catalyst according to claim 9, **characterized in that** the catalyst comprises MgO as isomerisation catalyst component and the at least one metathesis catalyst component in a ratio between 5:1 and 1:1, preferably in a ratio 4:1 and 2:1, most preferably in a ratio of 3:1.

11. Catalyst according to one of the claims 9 or 10, **characterized in that** the metathesis catalyst comprises oxides of metals of the 6^{th} and 7^{th} group of the PSE, in particular tungsten oxide, molybdenum oxide and/or a precursor thereof deposited on at least one inorganic carrier.

12. Catalyst according to one of the claims 9 to 11, **characterized in that** magnesium oxide according to one of the claims 1 to 6 is additionally arranged as a pre-bed upstream of the catalyst mixture of metathesis catalyst and isomerisation catalyst.

13. Catalyst according to claim 12, **characterized in that** the mass ratio of the pre-bed and the main catalyst bed being a mixture of metathesis catalyst and isomerisation catalyst is between is between 1:10 and 3:1, preferably between 1:6 and 2:1, most preferably between 1:4 and 1:2.

14. Catalyst according to one of the claims 9 to 13, **characterized in that,** said catalyst is activated in a process comprising the steps of
a) heating the catalyst in an inert gas atmosphere to a temperature between 300 °C and 500 °C, preferably 400 °C;
b) oxidizing the catalyst in an oxygen containing atmosphere at temperatures between 400 °C and 600 °C, preferably 400 °C and 550 °C;
c) reducing the catalyst in a hydrogen containing atmosphere at temperatures between 300 °C and 500 °C, preferably at 400 °C,
d) Heating of the catalyst in an inert gas atmosphere at temperatures between 400 °C and 600 °C, preferably 400 °C and 550 °C; and
e) subsequent cooling down the catalyst in an inert gas atmosphere.

15. Process for obtaining an olefin, in particular propene, by metathesis comprising the steps of
- feeding at least two olefins as starting material, to a reactor, in particular a fixed-bed reactor, comprising at least one catalyst according to one of claims 9 to 14;
- converting the at least two olefin gases at a pressure between 1 to 50 bar, in particular 10 to 30 bar, at a temperature between 100 and 600 °C, in particular between 250 and 500 °C to at least one new olefin.
